# EUROPEAN PATENT APPLICATION

(11) **EP 1 550 443 A1**
(43) Date of publication of application: **06.07.2005**
(21) Application number: 03797601.6
(22) Date of filing: 16.09.2003
(51) Int. Cl.: A61K 31/198, A61K 31/5517, A61K 45/00, A61P 25/00, A61P 25/18, A61P 43/00

(54) **COMPOSITION AGAINST STRESS-RELATED DISEASES**

(30) Priority: 18.09.2002 JP 2002271944
(71) Applicant: Ajinomoto Co., Inc., Tokyo 104-8315 (JP)
(72) Inventor: SMRIGA, Miroslav, Ajinomoto Co., Inc., Kawasaki-shi, Kanagawa 210-0801 (JP); TAKAHASHI, Kazuyoshi, Ajinomoto Co., Inc., Kawasaki-shi, Kanagawa 210-0801 (JP); MORINAGA, Yasushi, Ajinomoto Co., Inc., Kawasaki-shi, Kanagawa 210-0801 (JP)
(74) Representative: Nicholls, Kathryn Margaret
(86) International application number: PCT/JP2003/011754
(87) International publication number: WO 2004/026296

(57) **Abstract**

It is intended to provide an improved drug, compared with the existing ones, for stress-related diseases which comprises a combination of lysine wi than agent for stress-related diseases other than lysine, in particular, a drug for stress-related diseases having an enhanced effect (drug efficacy) and reduced side effects. The above drug, which can be employed in the form of a medicine (including a medicine for animals), a food, a drink or a feed, is highly useful in preventing, treating or ameliorating various stress-related diseases and preventing the progress of such diseases. The two ingredients as described above can be used either at the same time or at different points in different forms. It is also intended to provide a method of preventing and treating stress-related diseases, utilization of the above active ingredients in a preventive/remedy for stress-related diseases and so on.

## Description

### TECHNICAL FIELD

The present invention relates to a combination of lysine with a drug for stress-related diseases, other than the lysine, (drug exhibiting an anti-stress effect) administrable at the same time or separately as a novel composition (drug, drug for animal use, food, drink, feed, etc.) or drug (drug exhibiting an anti-stress effect) for treating stress-related diseases. The composition when used as a drug is very useful in treatment, prevention, prevention of development, or improvement of stress-related diseases in animals such as human.

The invention also relates to a method for treatment or prevention (including treatment, prevention, prevention of development, and improvement) of a stress-related disease and to the use of the above-mentioned active ingredient as a drug (including a drug for treatment, prevention, prevention of development, and improvement) for treatment or prevention of a stress-related disease.

### BACKGROUND ART

Stress affects various general organs such as circulatory organs and digestive organs to cause a stress-related disease such as depression, anxiety, gastro-duodenal ulcer, irritable bowel syndrome, bronchial asthma, hypertension, autonomic imbalance, and the like. In a case of depression, the employed basic therapeutic principles are: "taking a rest sufficiently", "using an appropriate anti-depressant", and "supportive psychotherapy like cognitive therapy". Though tricyclic or tetracyclic anti-depressants have so far been used in the drug therapy, they are accompanied by dry mouth, constipation, dysuria, disorder of eye control, and the like, making their use difficult. Recently, a selective serotonin reuptake inhibitor (SSRI) and selective serotonin/norepinephrine reuptake inhibitor (SNRI), which allow decrease of adverse reactions, have been proposed, but a side effect such as gastric mucosa disorder has been observed, and it is desired to avoid such a side effect, accordingly.

In another stress-related disease, irritable bowel syndrome, a certain drug therapy has been made in addition to psychotherapy and dietary therapy. In the drug therapy, however, a sufficient effect has not yet been attained, though an anti-cholinergic agent, anti-diarrheal agent or laxative as well as a minor tranquilizer has been used mainly for the relaxation of the disease condition (abdominal pain, diarrhea, constipation, etc.). Thus, a highly effective therapeutic agent is desired.

Thus, there are several problems in the treatment (therapy) of stress-related diseases with drugs, that is, (1) insufficient effect, and (2) occurrence of adverse reactions to reduce the usefulness. In order to solve or improve these problems, a variety of studies have been attempted up to now, but no satisfactory therapeutic method has been achieved.

In this situation, it is desired to develop an anti-stress agent in which the above-mentioned problems have been improved.

### DISCLOSURE OF INVENTION

### 1. Problem to be Solved by the Invention

The problem to be solved by the invention is to provide an anti-stress agent (a drug effective to a stress-related disease), for example, a drug in which the effect (drug efficacy) is enhanced more than that of the conventional drugs with lesser adverse reaction.

### 2. Means for Solving the Problems

The present inventors worked assiduously to solve the above problems and found that a composition prepared by blending one of amino acids, lysine, with an existing or coming anti-stress agent (drug for stress-related diseases) or a combination of lysine with the agent has an enhanced effect (drug efficacy) and reduced adverse reaction in comparison with the anti-stress agent *per se* containing no lysine. Thus, the invention was completed.

In this connection, recently, some of the present inventors found that one of essential amino acids, lysine, has anti-stress effect; this finding has been filed as a patent application PCT/JP02/02571. Therefore, since lysine corresponds to one of anti-stress agents, lysine and the another anti-stress agent are employed as active ingredients.

In the invention, it is considered that there is a possibility of lysine enhancing the effect of the drug used as a therapeutic for the above-mentioned diseases, resulting in decrease of the incidence of a side effect by reduction of a dose of the drug. Thus, the combined use of lysine increases synergistically or additively the effect of the anti-stress agents. In particular, among the drugs so far used in treatment of stress-related diseases, those of which the effect is insufficient in single use or the expected efficacy is reduced by their side effect, are expected to contribute to the treatment (therapy) of these stress-related diseases as medical or food stuffs since their usefulness is increased in combination with lysine.

That is, the invention relates to a composition for treating a stress-related disease which comprises at least lysine and another anti-stress agent other than lysine as active ingredients (a composition of the invention).

Lysine may be used in a form of the salts such as hydrochloride, glutamate. Lysine may be used in any form of the optical isomers including D-isomer, L-isomer and DL- isomer; preferably, the L-isomer is used in the view that it is naturally occurring. The subject to be treated with the anti-stress agent includes human (patient or a person who needs the prevention) as well as animal which has a stress-related disease or possibly suffering from such a disease, for example, domestic animal.

The above-mentioned composition may be used in a form of drugs (including animal drugs), food and drink, and feed.

As for the above-mentioned anti-stress agents, one or more of the following drugs (1) to (3) may be selected.
(1) Drugs acting on a neurotransmission system participating in the central and autonomic nervous system;
(2) Drugs acting on an immune system; and
(3) Drugs relieving a disease condition by a mechanism other than the neurotransmission system and immune system.

The above-mentioned anti-stress agents, one or more, may be selected from the following drugs.

Anti-depressants, anti-anxiety agents, agents for treating irritable bowel syndrome, agents for treating gastro-duodenal ulcer, agents for treating bronchial asthma, anti-hypertensive agents, agents for treating angina pectoris, anti-diabetics, and anti-rheumatism agents.

The above anti-depressants include selective serotonin reuptake inhibitors and selective serotonin/norepinephrine reuptake inhibitors; the above anti-anxiety agents include benzodiazepine derivatives, diphenylmethane derivatives and drugs acting on serotonin 5-HT1A receptors; the above agents for treating irritable bowel syndrome include anti-cholinergic agents, anti-diarrheal agents and laxatives; the above agents for treating gastro-duodenal ulcer include histamine H2 receptor antagonists, proton pump inhibitors and prostaglandin-type agents; the above agents for treating bronchial asthma include adrenaline β2 receptor agonists and anti-histaminics; the above anti-hypertensive agents include Ca-channel antagonists, angiotensin converting enzyme inhibitors, angiotensin II receptor antagonists and diuretics; the above agents for treating angina pectoris include organic nitric acid esters and adrenaline β receptor blockers; and the above anti-diabetics include insulin secretion promoters, insulin preparations and sulfonylureas.

The above-mentioned drugs are exemplified by anti-stress agents (drugs with an anti-stress effect) and can be used in treatment, prevention, improvement, and prevention of the development of such diseases.

There is no particular limitation in the ratio of the above-mentioned anti-stress agent (active ingredient) to lysine to be used. The ratio (by weight) of the anti-stress agent to lysine when calculated in the free form of lysine is preferably in the range of approximately 1:0.5 to 1:100000, more preferably 1:1 to 1:10000, and even more preferably 1:2 to 1:5000.

The above-mentioned composition of the invention comprises at least lysine and an anti-stress agent other than lysine (one or more of active ingredients), and further it may contain other ingredients as far as they do not disturb the purpose of the invention. For example, an amino acid other than lysine may be used as an additive. For example, arginine, glutamic acid, aspartic acid, and so on may be added.

In another embodiment of the invention, the anti-stress agent includes a combination of lysine with an anti-stress agent other than lysine which may be administered at the same time or separately (a combination of the invention).

In the same manner as in the above invention, lysine may be used in a form of salts such as the hydrochloride, glutamate, and the like. Lysine may be used in any form of the optical isomers including D- isomer, L- isomer and DL- isomer; preferably, the L-isomer is used since it is naturally occurring. The subj ect to be treated with the anti-stress agent includes humans as well as animals possibly suffering from stress-related diseases, for example, domestic animal.

The combination of the invention includes the above composition of the invention as a form for concurrent administration of the above two active ingredients. The description made in the above-mentioned composition of the invention all are applied to the present invention except for that of "composition" *per se*.

The combination of the invention may be used in any form including the above-mentioned drugs (including drugs for animals), food, drink, and feed. Inparticular, theanti-stress agents may be administered as a drug or a drug for animal use (single preparation); on the other hand, lysine may be given (administration, diet, or supply) in a form of drug, food and drink, or feed (single preparation of lysine, drink containing lysine, feed containing lysine, etc.), respectively at a different point of the time. In this operation, it is naturally possible that they may contain additional ingredients necessary for the drug (including a drug for animal use), food, drink, and feed, or they may further be processed if necessary.

In another embodiment, the invention provides a method for preventing or treating a stress-related disease which comprises ingesting or administering lysine and an anti-stress agent other than lysine to the living body (including treatment, prevention, prevention of development, improvement, etc.). Lysine may be used in a form of salts.

The ingestion or administration may be made by ingesting or administering the above-mentioned composition or in a form of the above-mentioned combination of the invention.

Still in another embodiment, the invention relates to the use of (or in preparation of) the drugs for preventing or treating stress-related diseases (including treatment, prevention, prevention of development, etc.) comprising lysine and an anti-stress agent other than lysine. Lysine may be in a form of salts.

The stress-related diseases are as described in the present description. As for the drugs for preventing or treating stress-related diseases, the above-mentioned composition or combination of the invention or one further containing the above-mentioned ingredients may be exemplified as a preferred embodiment.

### BRIEF DESCRIPTION OF DRAWINGS

Fig. 1 shows a combined effect of lysine and a minor tranquilizer alprazolam in an irritable bowel syndrome model (Example 1).
   * : significant in comparison with a control (p<0.05).
Fig. 2 shows a reducing effect of lysine for exacerbation of intragastric hemorrhage by SSRI in a gastric ulcer model (Example 2). SSRI: paroxetine
   * : significant in comparison with a control (p<0.05).

### MODE FOR CARRYING OUT THE INVENTION

The followings will explain the mode for carrying out the invention.

The subject of the invention as mentioned above can be used in a form of drugs (including drugs for animal use), food and drink, or feed. In this invention, the two active ingredients, i.e., lysine and an anti-stress agent (active ingredient), each can be used separately in a different form. Hereinafter, a representative formulation of the invention will be explained as an example of the products (pharmaceutical preparations) which contain the above two active ingredients in the same preparation, though it is a typical example and the invention is not limited thereto.

The stress-related diseases of the invention mean those caused by any stress (see: "Karada no Kagaku" (Body Science), 223: 58-61 (2002)), and will be explained in detail by the followings.

The autonomic nervous system and immune system are affected by stress to cause the following stress-related diseases. The diseases include depression or anxiety in a psychic nervous system; gastro-duodenal ulcer or irritable bowel syndrome in a digestive organ system; bronchial asthma or hyperventilation syndrome in a respiratory system; hypertension or angina pectoris in a circulatory system; diabetes mellitus in an endocrine system; migraine headache or autonomic imbalance in a neuromuscular system; and chronic articular rheumatism or collagen disease in an immune and allergy system.

The drugs used in these diseases can roughly be classified into three groups. The first group includes drugs acting on a neurotrasmission system participating in the central and autonomic nervous systems which are activated by stress per se. The second group includes drugs acting on an immune system altered by stress, and the third includes drugs improving (alleviating) the condition per se recognized in the respective diseases which are accompanied by alteration of the above neurotransmission system and immune system.

The effect of lysine on stress-related diseases is considered to occur as the effect on a serotonin nervous system and the modification to a benzodiazepine receptor (in house data) .

A drug of which the effect is expected to increase in combination with lysine, for example, a conventional drug or a drug which may be developed in future, includes preferably those of which the action mechanism is different from that of lysine. That is, it includes (1) a group of drugs acting on the neurotransmission system participating in the central and autonomic nervous system, which has the site of action different from that of a serotonin nervous system and benzodiazepine receptor in which lysine is possibly involved; (2) a group of drugs acting on an immune system; and (3) a group of drugs alleviating the condition based on a mechanism other than a neurotransmission system and immune system.

Concrete examples are: (1) a group of drugs acting on the neurotransmission system participating in the central and autonomic nervous system, including an SSRI which has recently been used as an anti-depressant, e.g., paroxetine and fluvoxamine, SNRI, e.g., milnacipran; benzodiazepine derivatives as anti-anxiety agents, e.g., diazepam, oxazepam and flutoprazepam; diphenylmethane derivatives, e.g., hydroxyzine; and serotonin 5-HT1A receptor agonists, e.g., tandospirone; as well as other drugs controlling neurotransmission (catecholamine, serotonin, acetylcholine, neuropeptides, etc.); adrenaline β-receptor blockers, e.g., propranolol, pindolol and timolol; adrenaline α1-receptor blockers, e.g., prazosin and bunazosin; adrenaline α,β-receptor blockers, e.g., labetalol; muscarine receptor antagonists, e.g., tiquizium and trospium; serotonin 5-HT3 receptor antagonists, e.g., alosetron; and serotonin 5-HT4 receptor agonists, e.g., tegarerod.
(2) A group of drugs acting on an immune system includes non-specific immunoactivators, e.g., lentinan and schizophyllan; interleukin production inhibitors, e.g. , cyclosporin, suplatast and tazanolast.
(3) A group of drugs alleviating the condition based on a mechanism other than a neurotransmission system and immune system includes drugs acting on gastro-duodenal ulcer, for example, histamine H2 receptor antagonists which directly inhibit the secretory mechanism of an aggressive factor, i.e. acid, e.g., famotidine, cimetidine, ranitidine and nizatidine; and proton pump inhibitors, e.g., lansoprazole, omeprazole and rabeprazole. Drugs for treating irritable bowel syndrome include those improving constipation, e.g., polycarbophil calcium, methylcellulose and ragnolose. The antihypertensive agents include Ca-channel antagonists which directly relaxes a vascular smooth muscle to decrease blood pressure, e.g., cilnidipine, amlodipine and efonidipine; angiotensin converting enzyme inhibitors which directly inhibit a hypertensive factor renin-angiotensin system, e.g., enalapril, captopril and temocapril; or angiotensin II receptor antagonists, e.g., losartan, valsartan and candesartan cilexetil. The drugs for treating bronchial asthma include adrenaline β2 receptor agonists, e.g., procaterol, trimethoquinol, hexoprenaline and salbutamol; the drugs for treating diabetes mellitus include those directly controlling insulin secretion, e.g., tolbutamide or nateglinide, glibenclamide and chlorpropamide.

In using the anti-stress agents as mentioned above, they may be given in a conventional way corresponding to the respective drugs; the dose of the active ingredient may be determined in the conventional way, which may be properly increased or decreased in consideration of the effect of the invention (dose of approximately 10-80%).

Therefore, it is considered that the occurrence of side effect caused by these drugs could be reduced by decreasing the dose with the effect enhanced by a combined use of lysine.

Lysine used in combination with a drug for treating stress-related diseases can be incorporated into the above pharmaceutical preparations. Of course, it may be used alone or as a pharmaceutical composition prepared by blending with a known pharmaceutically acceptable carriers, diluents, and the like. In such a case, there is no particular limitation in a way of administration, and it may be used as an oral preparation such as tablets, capsules, powders, granules, pills, and the like, or as a perenteral preparation such as injections, syrup, ointment, suppositories, and the like. The dose of lysine is variable depending on the subject to be administered, the route of administration, and conditions; it may be administered preferably at a daily dose of about 10 mg to 50 g, and more preferably about 100 mg to 20 g, once or in several divided doses a day regardless of its formulation whether lysine alone or combination drug.

The invention, as mentioned above, also relates to a method for preventing or treating stress-related diseases (including treatment, prevention, prevention of development, improvement, etc.), which comprises ingesting or administering lysine and an anti-stress agent other than lysine to the living body, or alternatively the use of (or in preparation of) a drug for preventing or treating stress-related diseases (including treatment, prevention, prevention of development, improvement, etc.), which comprises lysine and an anti-stress agent other than lysine. In these cases, lysine may be in a form of salts.

The present invention can readily be carried out based on the above explanation of the composition of the invention and a combination of the invention or the working examples as mentioned below, or if necessary by referring to a conventional known technology.

### PREFERRED MODE FOR CARRYING OUT THE INVENTION

The invention will be explained in more detail by the following examples, which are not intended to limit the invention.

### Example 1

### Effect of the combined use of alprazolam and lysine in an animal model for irritable bowel syndrome

### [Method]

An animal model of irritable bowel syndrome was prepared according to the method as described by Williams et al. (Williams, C.L., Villar, R.G., Peterson, J.M., Burks, T.F. (1988) Stress-induced changes in intestinal transit in the rat: a model for irritable bowel syndrome. Gastroenterol. 94: 611-621). That is, 7 weeks old Wistar male rats were put a cotton tape around their anterior limbs and kept in a bracket cage, and then the amount of feces excreted during 2 hours was measured. A test compound was orally administered 40 minutes before giving wrap-restraint stress. The following groups were examined: 1) control group; 2) a group to which lysine hydrochloride (1 g/kg) alone was given; 3) a group to which a minor tranquilizer alprazolam (10 mg/kg; Takeda Chem. Ind.) alone was given; and 4) a combination group to which lysine hydrochloride (1 g/kg) and alprazolam (10 mg/kg) were given. Each group consists of 10 animals.

### [Results]

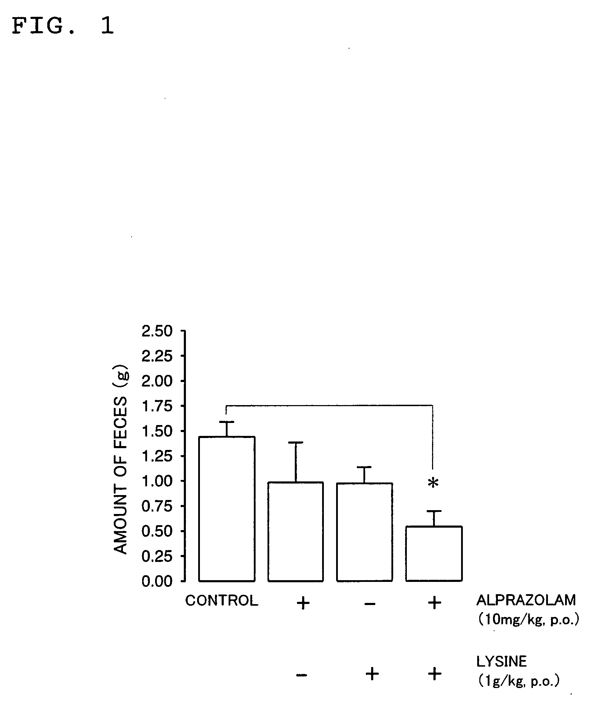

The amount of feces excreted 2 hours during which time wrap-restraint stress was laded was about 1.4 g; this value was significantly higher than that of no stress. In comparison with this increase induced by stress, no clear effect was observed in a group to which lysine hydrochloride or alprazolam alone was given. In a combination group to which lysine and alprazolam were given, however, the increase of feces was significantly inhibited (see Fig. 1) . That is, Fig. 1 indicates that lysine acts to increase the effect of the minor tranquilizer alprazolam.

### Example 2

### Effect of the combined use of SSRI and lysine in a model for gastric ulcer

### [Method]

A widely used model for gastric ulcer caused by water immersion under restraint was used. That is, rats were placed in a stress cage and immersed in water to the chest for 5 hours (at a temperature of 22-25 degree). The stomach was removed and the degree of intragastric hemorrhage was observed and the hemorrhage area was calculated using an NHI image software.

The test compound was evaluated as follows. Five-weeks old Wistar male rats were divided into 3 groups. The 1st group was bred with a normal powdered feed; the 2nd group wi th a powdered feed to which was added a selective serotonin reuptake inhibitor (SSRI)(paroxetine; Glaxo-SmithKline; 298 mg/kg diet) alone; and the 3rd group with a powdered feed containing SSRI and lysine hydrochloride (13.3 g/kg diet) to which was additionally added arginine (13.3 g/kg diet). Each group consisted of 10 animals. Rats could freely have a feed before they reached 14 weeks of age. Thereafter, no feed and water were given for 2 days, and on day 3, all of the rats were subjected to water-immersion restraint stress for 5 hours.

### [Results]

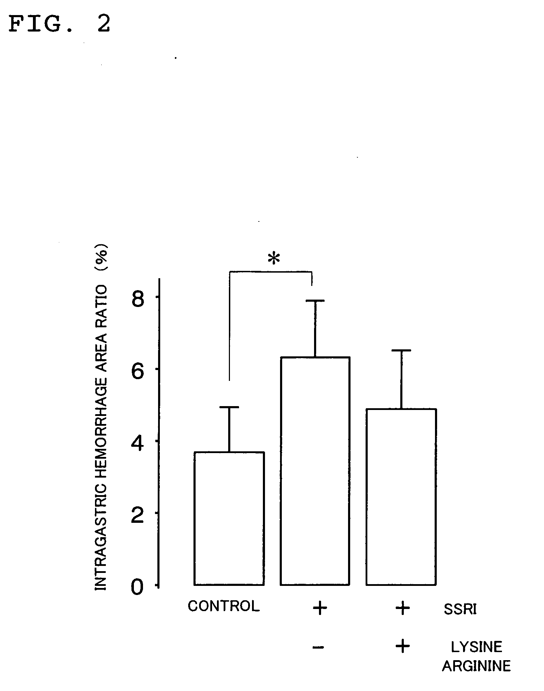

SSRI significantly exacerbated the intragastric hemorrhage, but no exacerbation by SSRI was observed in the combined use of lysine (see Fig. 2). That is, Fig. 2 indicates that exacerbation of the intragastric hemorrhage by SSRI is reduced.

From the above results, the effect of lysine combined with an anti-stress agent was confirmed.

### EFFECT OF THE INVENTION

As clearly seen from the above explanation, the invention provides a combination of lysine and an anti-stress agent, particularly a composition comprising the two active ingredients, for example, a drug such as a combination drug (including a drug for animal use). In comparison with a single preparation of anti-stress agent, particularly a conventional anti-stress agent, the combined use of lysine allows the improvement of pharmacological effect and side effect, and accordingly the products of the invention exhibit a very high efficacy. The invention further provides a method for preventing or treating stress-related diseases (including treatment, prevention, prevention of development, improvement, etc.) and use of the above-mentioned active ingredients in drugs for preventing or treating stress-related diseases (including treatment, prevention, prevention of development, improvement, etc.) . The invention can widely be applied in a field of drugs (including drugs for animal use), medication, food and feed, and is very useful in industries.

## Claims

1. A composition for stress-related diseases comprising lysine or a salt thereof and an anti-stress agent other than lysine as active ingredients.

2. A composition as claimed in Claim 1, being in a form of drugs, drugs for animal use, food, drink or feed.

3. A composition as claimed in Claim 1, wherein the anti-stress agent is selected from the following drugs (1) to (3) :
(1) drugs acting on a neurotransmission system participating in central and autonomic nervous systems;
(2) drugs acting on an immune system; and
(3) drugs relieving a disease condition by a mechanism other than the neurotransmission system and immune system.

4. A composition as claimed in any one of Claims 1 to 3, wherein the anti-stress agent is selected from the following drugs:
anti-depressants, anti-anxiety agents, agents for treating irritable bowel syndrome, agents for treating gastro-duodenal ulcer; agents for treating bronchial asthma, anti-hypertensive agents, agents for treating angina pectoris, anti-diabetics, and anti-rheumatism agents.

5. A composition as claimed in Claim 4, wherein the anti-depressant is selected from selective serotonin resorption inhibitors and selective serotonin/norepinephrine resorption inhibitors, and the anti-anxiety agent is selected from benzodiazepine derivatives, diphenylmethane derivatives and drugs acting on serotonin 5-HT1A receptors.

6. A composition as claimed in Claim 4, wherein the agents for treating irritable bowel syndrome is selected from anti-cholinergic agents, anti-diarrheal agents and laxatives, and the agents for treating gastro-duodenal ulcer is selected from histamine H2 receptor antagonists, proton pump inhibitors and prostaglandin-type agents.

7. A composition as claimed in Claim 4, wherein the agent for treating bronchial asthma is selected from adrenaline β2 receptor agonists and anti-histaminics; the antihypertensive agent is selected from Ca-channel antagonists, angiotensin converting enzyme inhibitors, angiotensin II receptor antagonists and diuretics; the agent for treating angina pectoris is selected from organic nitric acid esters and adrenaline β receptor blockers; and the anti-diabetic is selected from insulin secretion promoters, insulin preparations and sulfonylureas.

8. A composition as claimed in Claim 2, wherein the drug is an anti-stress agent.

9. A composition as claimed in any one of Claims 1 to 8,
wherein the ratio (by weight) of the anti-stress agent to lysine when calculated in a free form of lysine is in 1:0.5 to 1:100000.

10. A composition as claimed in Claim 1 or 2, which contains an amino acid other than lysine.

11. A combination of lysine or a salt thereof with an anti-stress agent other than lysine, each of which may be administered at the same time or separately as an anti-stress agent.

12. A method for preventing or treating stress-related diseases which comprises ingesting or administering lysine (or a salt thereof) and an anti-stress agent other than lysine into a living body.

13. A method as claimed in Claim 12, wherein the form for ingestion or administration is of a composition as claimed in any one of Claims 1 to 10 or of a combination as claimed in Claim 11.

14. Use of lysine and an anti-stress agent other than lysine for preventing or treating stress-related diseases.

15. Use as claimed in Claim 14, wherein the drug for preventing or treating stress-related diseases is in a form of a composition as claimed in any one of Claims 1 to 10 or of a combination as claimed in Claim 11.
